# EUROPEAN PATENT SPECIFICATION

(11) **EP 2 791 686 B1**
(45) Date of publication and mention of the grant of the patent: **15.02.2017**
(21) Application number: 12816091.8
(22) Date of filing: 12.12.2012
(51) Int. Cl.: G01N 33/68, G01N 33/564

(54) **TENASCIN-C AND USE THEREOF IN RHEUMATOID ARTHRITIS**
BIOMARKER UND VERWENDUNG DAVON
BIOMARQUEUR ET SON UTILISATION

(30) Priority: 12.12.2011 GB 201121280; 20.08.2012 GB 201214806
(43) Date of publication of application: 22.10.2014
(73) Proprietor: Oxford University Innovation Limited, Botley Oxford OX2 0JB (GB)
(72) Inventor: MIDWOOD, Kim Suzanne, Chesham BUCKS HP5 2JF (GB)
(74) Representative: Atkinson, Jennifer
(86) International application number: PCT/GB2012/053104
(87) International publication number: WO 2013/088140

(56) References cited:
- JP-A- 2004 138 489
- MIDWOOD KIM ET AL: "Tenascin-C is an endogenous activator of Toll-like receptor 4 that is essential for maintaining inflammation in arthritic joint disease", NATURE MEDICINE, NATURE PUBLISHING GROUP, NEW YORK, NY, US, vol. 15, no. 7, 1 July 2009 (2009-07-01), pages 774-780, XP002594138, ISSN: 1078-8956, DOI: 10.1038/NM.1987 [retrieved on 2009-06-28]
- PICCININI A M P ET AL: "Persistent expression of the pro-inflammatory extracellular matrix glycoprotein tenascin-C drives arthritic joint disease", INTERNATIONAL REVIEW OF EXPERIMENTAL PATHOLOGY, BLACKWELL SCIENTIFIC, OXFORD, GB, vol. 92, no. 3, 1 June 2011 (2011-06-01), page A30, XP009169209, ISSN: 0959-9673 [retrieved on 2011-05-14]
- MASAHIRO HASEGAWA ET AL: "Expression of large tenascin-C splice variants in synovial fluid of patients with rheumatoid arthritis", JOURNAL OF ORTHOPAEDIC RESEARCH, vol. 25, no. 5, 1 May 2007 (2007-05-01), pages 563-568, XP055062003, ISSN: 0736-0266, DOI: 10.1002/jor.20366
- SCHENK SUSANNE ET AL: "Tenascin-C in serum: A questionable tumor marker", INTERNATIONAL JOURNAL OF CANCER, JOHN WILEY & SONS, INC, NEW YORK, NY; US, vol. 61, no. 4, 1 January 1995 (1995-01-01), pages 443-449, XP002501349, ISSN: 0020-7136, DOI: 10.1002/IJC.2910610402
- KIM S. MIDWOOD ET AL: "The role of tenascin-C in tissue injury and tumorigenesis", JOURNAL OF CELL COMMUNICATION AND SIGNALING, vol. 3, no. 3-4, 1 December 2009 (2009-12-01), pages 287-310, XP055000224, ISSN: 1873-9601, DOI: 10.1007/s12079-009-0075-1
- PICCININI ANNA M ET AL: "Endogenous control of immunity against infection: tenascin-C regulates TLR4-mediated inflammation via microRNA-155.", CELL REPORTS 25 OCT 2012, vol. 2, no. 4, 25 October 2012 (2012-10-25), pages 914-926, XP002696661, ISSN: 2211-1247
- THERESA H PAGE ET AL: "Raised circulating tenascin-C in rheumatoid arthritis", ARTHRITIS RESEARCH AND THERAPY, BIOMED CENTRAL, LONDON, GB, vol. 14, no. 6, 29 November 2012 (2012-11-29), page R260, XP021138358, ISSN: 1478-6354, DOI: 10.1186/AR4105
- MICHAELA RUHMANN ET AL: "Endogenous activation of adaptive immunity: Tenascin-C drives interleukin-17 synthesis in murine arthritic joint disease", ARTHRITIS & RHEUMATISM, vol. 64, no. 7, 1 July 2012 (2012-07-01), pages 2179-2190, XP055061293, ISSN: 0004-3591, DOI: 10.1002/art.34401

## Description

The present invention relates to the use of tenascin-C as a biomarker for inflammatory disorders, and in particular as a serum biomarker for rheumatoid arthritis. In particular, the invention relates to the use of tenascin-C as a biomarker for erosive rheumatoid arthritis.

Tenascin-C is a proinflammatory extracellular matrix glycoprotein (Midwood et al (2009) Nat. Med. 15: 774-780), that induces inflammatory cytokine synthesis in primary human macrophages and synovial fibroblasts by activation of the pattern recognition receptor, TLR4. Tenascin-C is a large hexameric protein of 1.5 million Da. High levels of tenascin-C have been found at sites of inflammation, for example in the synovial fluid of rheumatoid arthritis patients and in tumor stroma.

Rheumatoid arthritis is a chronic disease characterized by prolonged inflammation, swelling and pain of multiple joints. With time, the chronic inflammation leads to bone destruction within the joints and to progressive disability. One prominent hallmark of rheumatoid arthritis is wide variability in its clinical presentation. This variability extends to the level of pain, number of swollen joints and extent of joint deformity. Similarly, the response of patients with rheumatoid arthritis to any specific medical therapy also varies widely, from near elimination of disease signs and symptoms in some patients, to almost complete unresponsiveness in others.

The current diagnostic approach for rheumatoid arthritis was established by the American College of Rheumatology and is composed of the following items:
1) morning stiffness lasting more than 1 hour (mainly in fingers);
2) swelling in more than 3 joints;
3) swelling in joints of hand (wrists, metacarpophalangeal joints and proximal interphalangeal joints);
4) swelling in symmetrical joints (right and left joints);
5) abnormal findings of radiography of hand;
6) subcutaneous nodules; and
7) test positive for rheumatism by a blood test for CRP (C-reactive protein) or anti-CCP (anti-cyclic citrullinated peptide). The case which satisfies more than 4 items is diagnosed as rheumatoid arthritis.

It is an object of the present invention to provide an alternative marker, a diagnostic agent, and a detecting method that may be conveniently used for the identification of rheumatoid arthritis, and in particular for the identification of erosive rheumatoid arthritis.

Erosive rheumatoid arthritis is a major cause of disability in patients with rheumatoid arthritis, and is characterised by bone loss and local erosion of articular bone.
Determination of bone erosion is almost exclusively based on radiographic findings, ultrasound and doplar studies, because direct assessment of these lesions through biopsy is only rarely practical. The term 'bone erosion' describes loss of mineralized tissue at juxta-articular sites, which is commonly associated with a break in the cortical lining. Bone erosion starts early in disease and progresses most rapidly during the first year, thus the ability to rapidly detect and treat erosive rheumatoid arthritis would be of great benefit.

JP2004138489 is a patent publication that describes an arthritis diagnostic kit including an antibody for recognizing a tenascin C polymer splicing variant.

Midwood et al (2009. Nature Medicine, Vol 15:7, pp 774-780) describes that tenascin-C is an endogenous activator of Toll-like receptor 4 that is essential for maintaining inflammation in arthritic joint disease.

Piccinini et al (2011. International Review of Experimental Pathology, Vol. 92:3, page A30) discusses that persistent expression of the pro-inflammatory extracellular matrix glycoprotein tenascin-C drives arthritic joint disease.

Masahiro Hasegawa et al (2007. Journal of Orthopaedic Research, Vol. 25:5, pp 563-568) describes expression of large tenascin-C splice variants in synovial fluid of patients with rheumatoid arthritis.

Schenk et al (1995. International Journal of Cancer, Vol. 61:4, pp 443-449) describes tenascin-C in serum as a questionable tumour marker.

According to a first aspect, the invention provides a method of determining the rheumatoid arthritis status of a subject comprising the steps of:
(a) determining the level of tenascin-C in a blood sample from said subject; and
(b) comparing the level of tenascin-C determined in step (a) with one or more reference values.

According to another aspect of the invention, there is provided a method in accordance with claim 2 herein.

According to another aspect of the invention, there is provided a method in accordance with claim 3 herein.

The rheumatoid arthritis may be erosive rheumatoid arthritis.

Erosive rheumatoid arthritis is defined as rheumatoid arthritis with at least one erosive lesion in the articular bone, preferably there are at least five lesions, preferably there are at least ten lesions, preferably there are at least 20 lesions. The higher the number of lesions the more severe the erosive rheumatoid arthritis.

The method of the invention may be used to determine the erosive rheumatoid arthritis status of a subject who has already been diagnosed as having rheumatoid arthritis.
Preferably the method of the invention provides an easy, non-invasive and cheap way to look at the erosive rheumatoid arthritis status of a patient with rheumatoid arthritis.

The sample is a blood sample, such as a whole blood sample, plasma or serum. Preferably the sample is a serum sample.

Preferably the method is used to determine whether or not a subject has erosive rheumatoid arthritis.

Tenascin-C is a novel serum biomarker for inflammatory disorders, and rheumatoid arthritis in particular, and more specifically erosive rheumatic arthritis.

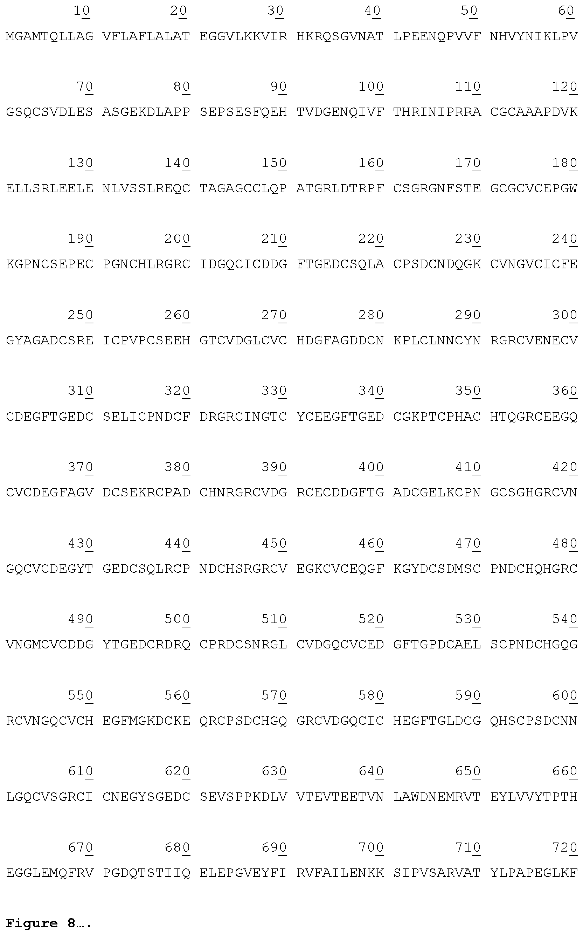

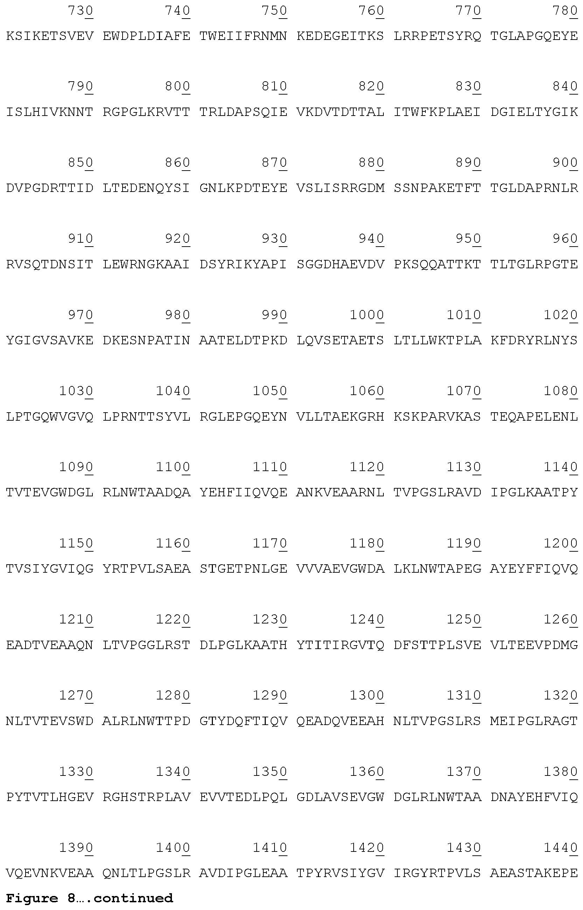

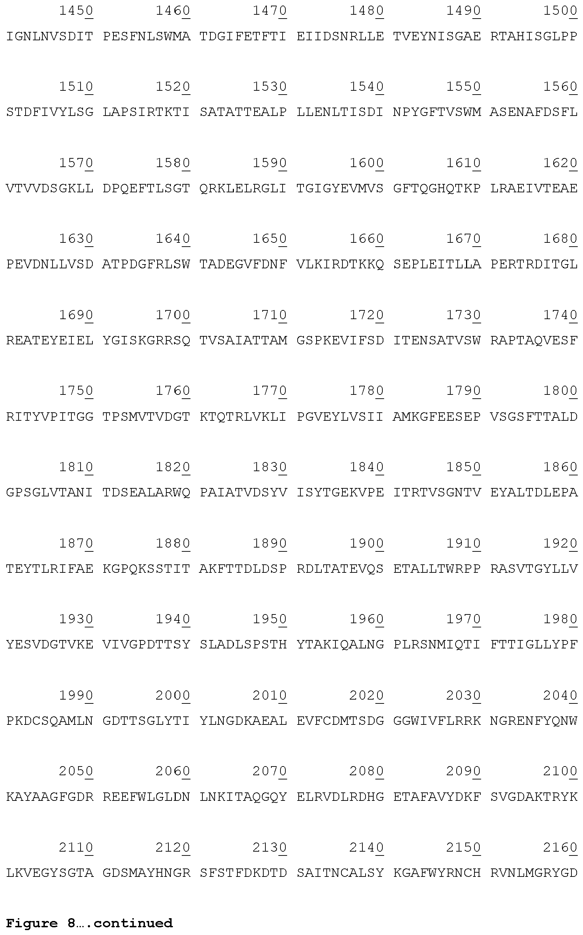

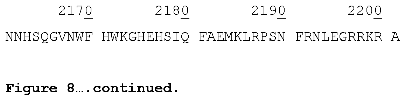

The protein sequence of human tenascin C can be found on GenBank using the accession number P24821 and the amino acid sequence is given in Figure 8.

In the method of the invention all forms of tenascin C may be measured. Preferably at least a 320kDa isoform of tenascin C is measured. Preferably only a 320kDa isoform of tenascin C is measured.

The phrase "inflammatory disorder status" includes any distinguishable manifestation of the inflammatory disorder. For example, inflammatory disorder status includes, without limitation, the presence or absence of the inflammatory disorder, the risk of developing the inflammatory disorder, the stage of the inflammatory disorder, the progression of the inflammatory disorder, and the effectiveness or response of a subject to a treatment for the inflammatory disorder.

The method of the invention may be used, for example, for any one or more of the following: to diagnose an inflammatory disorder in a subject; to assess the chance of a subject developing an inflammatory disorder; to advise on the prognosis for a subject with an inflammatory disorder; to monitor disease progression; and to monitor effectiveness or response of a subject to a treatment for an inflammatory disorder.

Preferably the method allows the diagnosis of an inflammatory disorder in a subject from the analysis of the level of tenascin-C in a sample provided by the subject.

The phrase "rheumatoid arthritis status" includes any distinguishable manifestation of the disease. For example, rheumatoid arthritis status includes, without limitation, the presence or absence of rheumatoid arthritis, the risk of developing rheumatoid arthritis, the stage of rheumatoid arthritis, the progression of rheumatoid arthritis, and the effectiveness or response of a subject to a treatment for rheumatoid arthritis. In a preferred embodiment rheumatoid arthritis status refers to the absence or presence of erosive rheumatoid arthritis.

The method of the invention may be used, for example, for any one or more of the following: to diagnose rheumatoid arthritis status in a subject, in particular erosive rheumatoid arthritis; to assess the chance of a subject developing rheumatoid arthritis, and in particular erosive rheumatoid arthritis; to advise on the prognosis for a subject with rheumatoid arthritis, and in particular erosive rheumatoid arthritis; to monitor disease progression, and in particular erosive rheumatoid arthritis progression; and to monitor effectiveness or response of a subject to a treatment for rheumatoid arthritis, and in particular erosive rheumatoid arthritis.

Preferably the method allows the diagnosis of rheumatoid arthritis, and in particular erosive rheumatoid arthritis, in a subject from the analysis of the level of tenascin-C in a sample provided by the subject.

The level of the tenascin-C present in a sample may be determined by any suitable assay, which may comprise the use of any of the group comprising immunoassays, spectrometry, western blot, ELISA, immunoprecipitation, slot or dot blot assay, isoelectric focussing, SDS-PAGE and antibody microarray immunohistological staining, radio immuno assay (RIA), fluoroimmunoassay, an immunoassay using an avidin-biotin or streptoavidin-biotin system, etc or combinations thereof. These methods are well known to persons skilled in the art.

Alternatively, the level of tenascin-C may be measured by determining the tenascin-C mPvNA levels in a sample. The mRNA levels could be measured by PCT or any other suitable technique.

Preferably the reference value, to which the determined levels of tenascin-C are compared, is the level of tenascin-C observed in one or more subjects that do not have any detectable inflammatory disorder, such as rheumatoid arthritis, or any clinical symptoms of an inflammatory disorder, such as rheumatoid arthritis, and have so called "normal values" of the biomarker tenascin-C.

Preferably an about 50% or more increase in tenascin-C in a sample, compared to the level in a normal tissue sample, is diagnostic of an inflammatory disorder, for example rheumatoid arthritis, and in particular of erosive rheumatoid arthritis.

Preferably a level of more than 3 1ng, preferably more than 33ng, of tenascin-C per ml of serum or plasma is diagnostic or at least indicative of rheumatoid arthritis, and in particular of erosive rheumatoid arthritis. Preferably the level of tenascin-C in combination with other markers allows a diagnosis of rheumatoid arthritis, and in particular of erosive rheumatoid arthritis.

Alternatively, the reference value may be a previous value obtained for a specific subject. This kind of reference value may be used if the method is to be used to monitor progression of disease or to monitor the response of a subject to a particular treatment.

Preferably, an increase in the level of tenascin-C compared to a normal/non-diseased reference level is indicative, or diagnostic, of rheumatoid arthritis, and erosive rheumatoid arthritis in particular.

The level of tenascin-C may be used to stratify patients with rheumatoid arthritis to those with erosive rheumatoid arthritis and those without.

The method of the invention may also be used to monitor rheumatoid arthritis progression, and erosive rheumatoid arthritis progression in particular, and/or to monitor the efficacy of treatments administered to a subject. This may be achieved by analysing samples taken from a subject at various time points following initial diagnosis and monitoring the changes in the levels of tenascin-C and comparing these levels to normal and/or reference values. In this case reference levels may include the initial levels of the biomarker in the subject, or the levels of the biomarker in the subject when they were last tested, or both.

The method of the invention may also be used to determine the appropriate treatment for a subject. The method may be used to offer personalised medicine solutions. In one embodiment, an increased level of tenascin-C in a sample, sufficient to result in a diagnosis of an inflammatory condition such as rheumatoid arthritis, may be used to indicate that it is not appropriate to use an anti-TNF drug as the subject is not likely to respond. It may be more appropriate to give an alternative therapy, such as an anti- IL 17 therapy; a T-cell co-stimulation modulator (such as Orencia™ - abatacept): an interleukin-6 (IL-6) inhibitor (such as Actemra™ - tocilizumab); an anti-CD20 antibody (such as Rituxan™ - rituxumab; or a B cell activating factor (such as anti-BAFF). Other alternative therapies include inhibitors of j anus kinase (JAK) (such as Tofacitinib™) and inhibitors of spleen tyrosine kinase (Syk) (such as Fostamatinib™).

In one embodiment the invention provides a method for determining whether anti-TNF therapy is an appropriate treatment for a patient with rheumatoid arthritis. More specifically, if a patient with rheumatoid arthritis has a tenascin-C level in their plasma or serum of at least 3 1ng, or at least 33ng, per ml of plasma or serum then they are unlikely to respond to anti-TNF therapy.

Preferably the method of the invention is carried out in vitro.

The subject may be mammal, and is preferably a human, but may alternatively be a monkey, ape, cat, dog, cow, horse, rabbit or rodent.

Preferably the sample is a serum sample or a plasma sample. Preferably the level of tenascin-C in the sample is at least 3 1ng, or at least 33ng, per ml of sample. Preferably the reference value is the value of tenascin-C in a plasma or a serum sample from a subject who does not have rheumatoid arthritis. The skilled man will appreciate that all the preferred features referred to above may also apply to this and every aspect of the invention.

A kit may be used in determining the inflammatory disorder status of a subject comprising at least one agent for determining the level of tenascin-C in a sample provided by the subject.

The kit may be for use in determining the rheumatoid arthritis status, and in particular the erosive rheumatoid arthritis status, of a subject comprising at least one agent for determining the level of tenascin-C in a sample provided by the subject.

The agent may be an antibody.

The kit may comprise instructions for suitable operational parameters in the form of a label or separate insert. The instructions may inform a consumer about how to collect the sample.

The kit may comprise one or more tenascin-C samples to be used as standard(s) for calibration and comparison. The kit may also comprise instructions to compare the level of tenascin-C detected in a sample with a calibration sample or chart. The kit may also include instructions indicating what level of tenascin-C is diagnostic of rheumatoid arthritis, and of erosive rheumatoid arthritis in particular.

According to a yet further aspect, the invention provides the use in accordance with claim 10 herein.

The use may be as a means of assessing the erosive rheumatoid arthritis status in an individual.

The skilled man will appreciate that preferred features of any one embodiment and/or aspect of the invention may be applied to all other embodiments and/or aspects of the invention.

The present invention will be further described in more detail, by way of example only, with reference to the following figures in which:

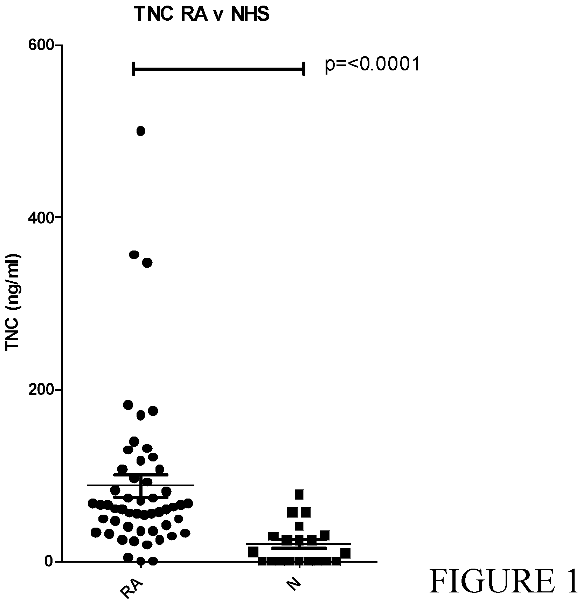

**Figure 1** **-** illustrates that tenascin-C levels are significantly increased in patients with rheumatoid arthritis. 52 patients were considered in this study, and 86.6% of those with rheumatoid arthritis were shown to have abnormally high (significantly increased) levels of tenascin-C. The results demonstrate that the mean tenascin-C value for rheumatoid arthritis patients is 87.87ng/ml of serum, and for the normal population the mean tenascin-C value is 20.34ng/ml of serum. The 95% confidence limit for the patients with rheumatoid arthritis is 31ng/ml, and thus it is concluded that levels of tenascin- C of above 3 1ng/ml serum are diagnostic of rheumatoid arthritis.

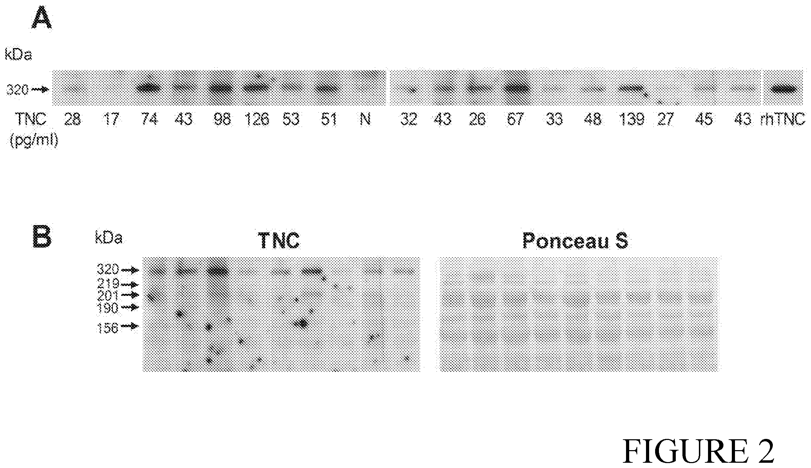

**Figure 2** **-** is a Western blot showing circulating levels of tenascin C in patients with rheumatoid arthritis (RA). Figure 2A shows that in baseline samples from RA patients one predominant TNC band of 320 kDa was observed (n=18). Corresponding TNC levels detected in the same samples by ELISA are shown in pg/ml under the blot. Low levels of TNC were detected in healthy control plasma (N). rhTNC= 0.05 µg human recombinant TNC. Figure 2B illustrates that in addition to the major band detected at 320kDa, bands of MW 219, 201, 190 and 156 kDa were present in the plasma of some RA patients upon western blotting with anti tenascin-C antibodies (TNC). Ponceau S staining of the same membrane shows equivalent protein loading for each RA sample.

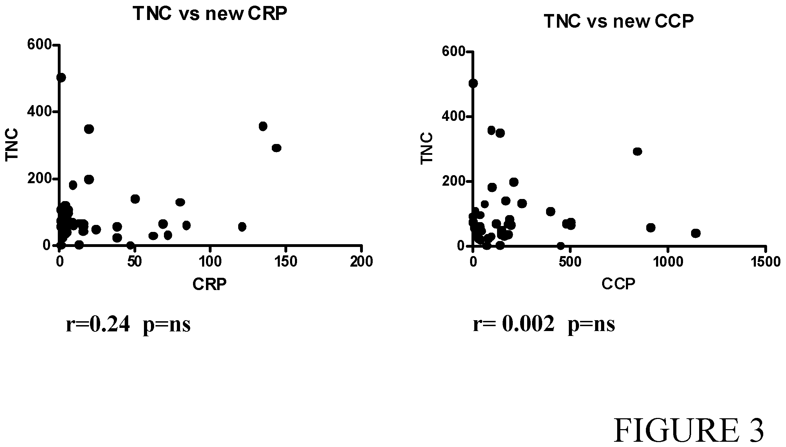

**Figure 3** **-** demonstrates that there is no correlation between tenascin-C levels and the levels of currently used CRP and anti-CCP markers for arthritis. Thus demonstrating tenascin-C to be an alternative and improved biomaker, in particular for rheumatoid arthritis, and erosive rheumatoid arthritis specifically. 52 patients were considered in this study, and serum levels of tenascin-C compared to serum levels of CRP and anti-CCP. There was no relationship between levels of TNC with either CRP and anti-CCP indicating that tenascin-C is a unique biomarker of rheumatoid arthritis.

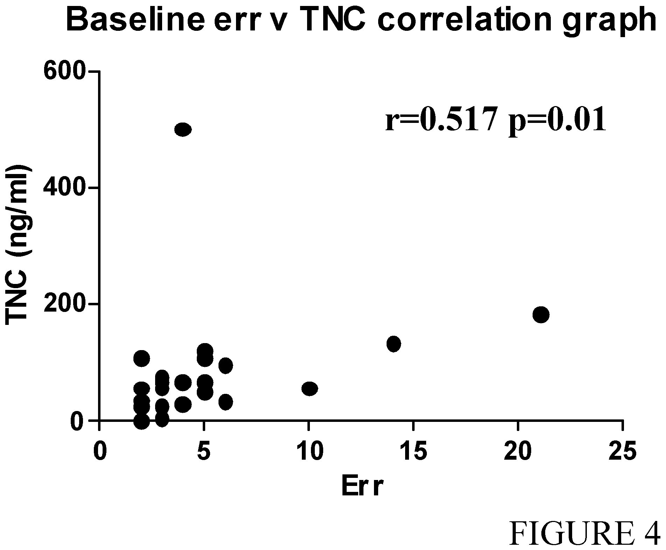

**Figure 4** **-** illustrates that there is a correlation between the level of tenascin-C expression in the serum of a patient with rheumatoid arthritis and the degree of erosion observed in the joints of a patient - the x axis represents the number of bone erosions observed. The data presented shows that the level of tenascin-C in serum is indicative of the presence of bone erosions in the joint and that the higher the tenascin-C serum levels the more erosion is observed.

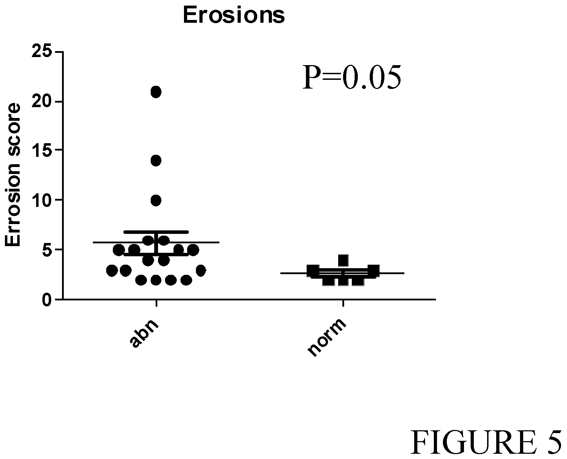

**Figure 5** **-** illustrates that when patients with rheumatoid arthritis are divided into those with abnormal levels of tenascin-C in the serum (that is, greater than 31ng/ml) and those with normal levels of tenascin-C (that is with tenascin-C levels in the serum of 0-3 1ng/ml) the patients with abnormal tenascin-C levels have a statistically increased erosion score. Thus indicating that the tenascin-C level in the serum is a good biomarker for erosive rheumatoid arthritis.

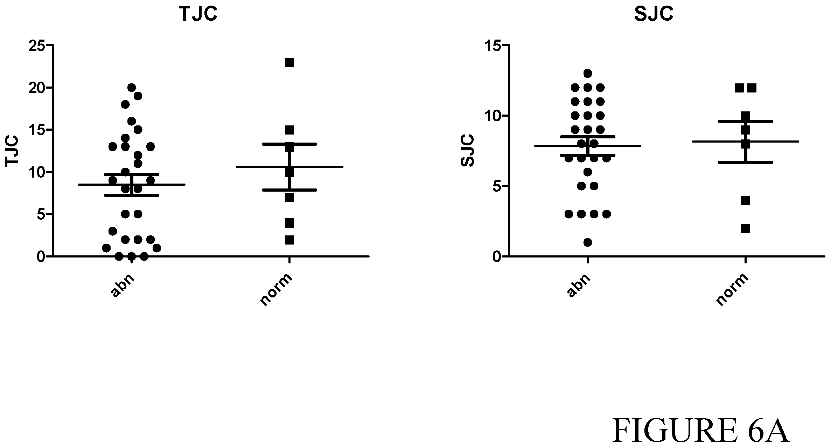

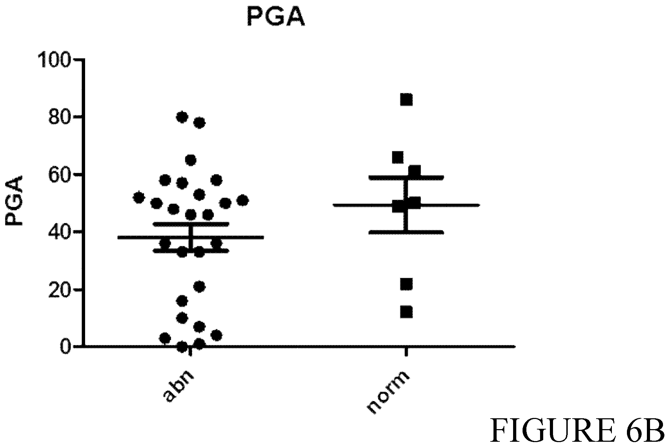

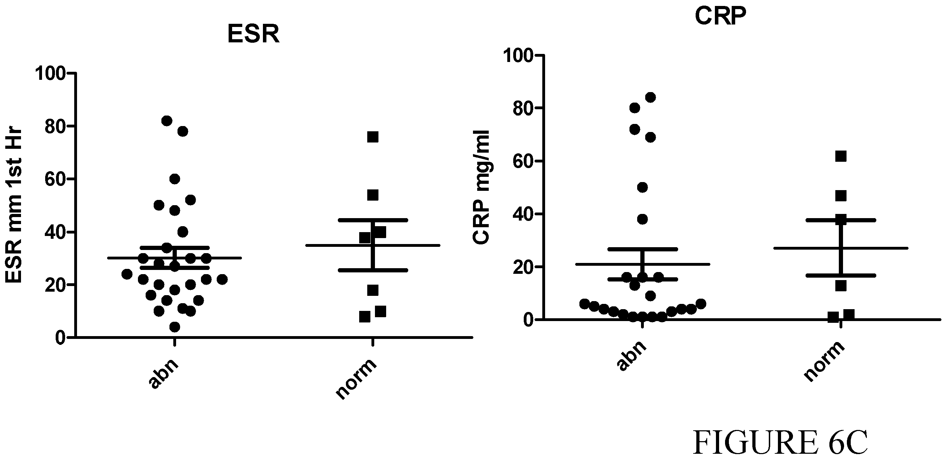

**Figures 6A - 6C** **-** demonstrate that there is no correlation between tenascin-C levels and tender and swollen joint count (counted by the GP) (Figure 6A), or with the global assessment score (Physician global assessment. On a scale 1 - 10 how does the doctor rate the activity of the patients disease) (Figure 6B) or the biochemical marker CRP or with the erythrocyte sedimentation rate (ESR) (Figure 6C). CRP is an acute phase protein that is elevated in inflammation, and is a non-specific maker of inflammation, levels are measured by ELISA. The ESR is the rate at which red blood cells sediment in a period of 1 hour, and is a non-specific measure of inflammation. To perform the test, anticoagulated blood is placed in an upright tube, known as a Westergren tube, and the rate at which the red blood cells fall is measured and reported in mm/h. The ESR is governed by the balance between pro-sedimentation factors, mainly fibrinogen, and those factors resisting sedimentation, namely the negative charge of the erythrocytes (zeta potential). When an inflammatory process is present, the high proportion of fibrinogen in the blood causes red blood cells to stick to each other.

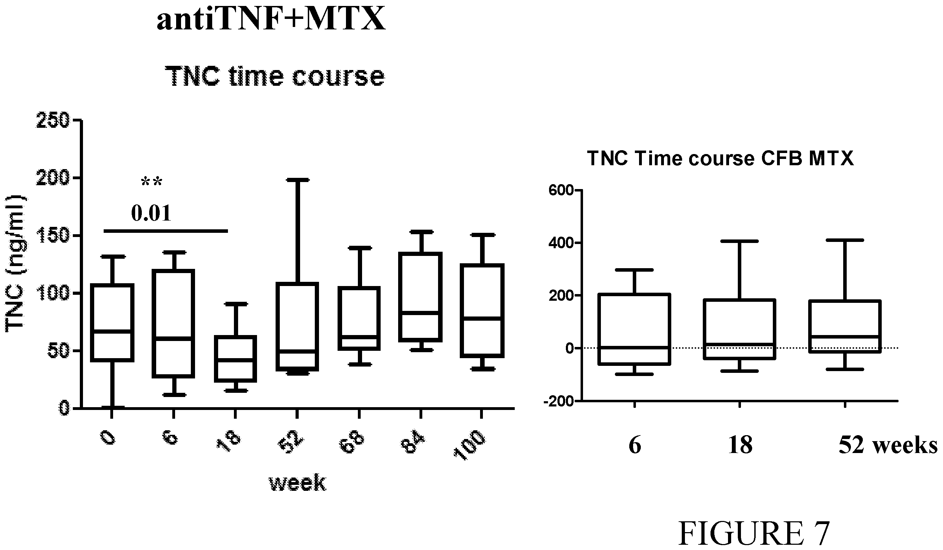

**Figure 7**- demonstrates the effect of the anti-TNF therapy Infliximab™ and methotrexate (MTX) on the tenascin-C levels in seven patients with rheumatoid arthritis. The results demonstrate that these therapies have very little long term effect on tenascin-C.
**Figure 8** **-** details the amino acid sequence of human tenascin C.

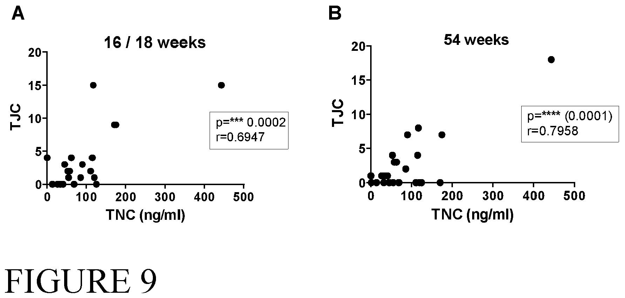

**Figure 9** **-** demonstrates that tenascin-C level at baseline is predictive of future tender joint count in RA patients at both 16- 18 weeks and 54 weeks after commencing infliximab (anti-TNF) therapy. Serum from RA patients in cohort A at baseline (entry into the trial) was analysed for tenascin-C level by ELISA. Tenascin-C levels are shown with practitioner determined tender joint counts in these patients at 16- 18 weeks (A) and 54 weeks (B) after receiving Infliximab therapy. Significance was determined by spearman rank correlation analysis. Cohort A includes patients with a diagnosis of erosive RA according to the ACR 1987 criteria, with symptoms of 0.5 to 3 years duration. Cohort B includes patients with a diagnosis of RA with disease activity scored at a moderate level or higher.

### Assessment of tenascin-C levels in patients with rheumatoid arthritis in comparison with in normal/control subjects

Serum was obtained from the blood of patients with rheumatoid arthritis and from normal subject and stored at -80°C until used. The sera was thawed on ice on the day of analysis and diluted typically 1 : 50 in EIA buffer ( 1 % BSA, 0.05% Tween 20 in PBS) for use in ELISA. ELISA kits were purchased from IBL (Cat No. 2775 1) and used to detect the high molecular weight variant of human tenascin-C. I OOµI of diluted sera was incubated on ELISA plates pre-coated with non-labelled capture anti-tenascin-C antibody, in duplicate at 37°C for 60 mins. The plates were washed 7 times (in 0.001 % Tween 20/PBS) and HRP-labelled anti-human tenascin-C antibody was added ( I OOµI) to each well. The wells were then incubated for 30mins at 4°C, and then washed 9 times with 0.001 % Tween 20/PBS . I OOµI Chromogen (supplied TMB solution) was then added to each well and incubated in the dark for 30 mins or until colour change judged to be sufficient. The reaction was stopped by the addition of I OOµI IN H2S04. The colour change was read at 450nm using a multiscan plate analyser, and the concentration of tenascin-C was calculated using Ascent version 2.6. Using standard curve generated from the tenascin-C supplied with ELISA plate (24 - 0.38 ng/ml) as a standard.

The above ELISA was performed on serum samples obtained from 52 patients with rheumatoid arthritis and from 20 normal control subjects who showed no signs of rheumatoid arthritis. The results are shown in Figure 1 and demonstrate a significantly higher level of tenascin-C in the rheumatoid arthritis patients, and allows the conclusion to be drawn that a level of tenascin-C greater than 3 Ing/ml, or greater than 33ng/ml, of serum is diagnostic of rheumatoid arthritis, and erosive rheumatoid arthritis in particular.

Western blotting revealed that the major form of tenascin-C present in rheumatoid arthritis patient samples has a mass of 320kDa, (Figure 2A) but minor, smaller, forms of tenascin-C were also observed in some patients (Figure 2B).

The Western blot data in Figure 2 was obtained using the following method. I µ 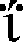 of serum or plasma was electrophoresed on 4- 12% Bis-Tris pre-cast polyacrylamide gels (Invitrogen, Life Technologies, Paisley, UK). Proteins were transferred onto nitrocellulose membranes (Amersham, GE Healthcare, Chalfont, UK) and visualised using Ponceau S stain (Sigma Aldrich, Gillingham, UK) . After washing with TBS/0.01 % Tween to remove stain, membranes were blocked in 5% BSA/TBS-Tween for lh at room temperature and incubated with primary antibody recognising the N-terminal region of human TNC (MAB 1908, Merck Millipore, Watford UK) at 1 : 1000 dilution for lh at 37°C. Horseradish peroxidase-conjugated anti-mouse IgG (Dako, Ely, UK) was used as a secondary antibody at a dilution of 1 : 3000. Bound antibody was detected using the enhanced chemiluminescence kit (Amersham, GE Healthcare) and visualized on Super RX medical X-ray film (Fuji, Japan

### Correlation between tenascin-C levels in patients with rheumatoid arthritis and the level of bone erosion

In order to demonstrate the correlation between the level of tenascin-C in a patient's serum and erosive rheumatoid arthritis, the level of erosion in the rheumatoid arthritis patients was compared to the level of circulating tenascin-C in the serum.

Erosion was measured using ultrasound and power doplar imaging as described by Taylor PC in Rheumatology (Oxford). 2005 Jun;44(6): 721 -8. Epub 2005 Jan 11. Review.

As can be seen in Figure 4 when the erosion score is plotted against the tenascin-C levels a significant correlation is observed.

Figure 5 goes on to demonstrate an even stronger correlation with erosive rheumatoid arthritis by comparing the erosion score of patients with rheumatoid arthritis who have abnormal levels of tenascin-C in the serum (that is, greater than 3 1ng/ml) with those of patients with rheumatoid arthritis who have normal levels of tenascin-C (that is with tenascin-C levels in the serum of 0-3 1ng/ml). The patients with abnormal tenascin-C levels were shown to have a statistically increased erosion score. Thus indicating that tenascin-C levels in the serum is good biomarker of erosive rheumatoid arthritis.

Furthermore, no significant correlation with other measures of rheumatoid arthritis and tenascin-C levels was observed (Figures 6A-6C), further indicating tenascin-C levels in serum to be a good biomarker for rheumatoid arthritis, and in particular for erosive rheumatoid arthritis.

### The effect of treatments for rheumatoid arthritis on tenascin-C levels

As illustrated in Figure 7, currently used treatments for rheumatoid arthritis do not appear to have any effect on tenascin-C levels.

### High serum tenascin-C is predictive of unresolved joint tenderness in infliximab treated patients

Despite the major advances provided by therapy with anti-TNF agents such as infliximab, a significant proportion of rheumatoid arthritis patients treated with anti-TNF agents do not respond and continue to acquire joint erosions and increased DAS28 scores. In the light of the correlation of tenascin-C levels with erosion scores and the effect of infliximab on circulating tenascin-C levels, studies were undertaken to determine whether the level of tenascin-C served as a predictor of future disease progression in infliximab treated patients. There was no relationship between baseline level of tenascin-C or change in tenascin-C level at weeks 18 or 52 after commencement of therapy and response to infliximab. However, a significant correlation was seen between the level of tenascin-C at baseline and the subsequent TJC score in infliximab treated patients at both 16-18 and 54 weeks after the start of treatment (Figure 9). Thus, these data suggest that patients with higher levels of tenascin-C before the initiation of infliximab therapy are more likely to be associated with unresolved joint tenderness despite infliximab therapy.

The results indicate that the level of tenascin-C in serum before beginning anti-TNF treatment acts as a predictor of tender joint count in a patient as far as one year in advance and allows patients that are likely to benefit from anti-TNF treatment to be distinguished from those who are not likely to respond.

Furthermore the results presented in Figure 2 indicate that the predominant variant of tenascin-C in the serum of patients with rheumatoid arthritis, and erosive rheumatoid arthritis in particular, is an isoform 320kDa in mass, which constitutes a 'large isoform' possessing. Tenascin-C is a large, multimodular molecule. It comprises a number of distinct domains including an assembly domain, a series of 14 and a half epidermal growth factor-like repeats, a series of up to 17 fibronectin type Ill-like repeats (TNIII), and a fibrinogen-like globe. Tenascin-C is encoded by a single gene that is alternatively spliced to create monomers ranging in size from 190-320 kDa. This occurs specifically in the TNIII domains; 8 of which are constitutively expressed (TNIII 1 -8) and 9 of which can be alternatively spliced (A 1 -4, B, AD2, AD 1 , C and D).

## Claims

1. A method of determining the rheumatoid arthritis status of a subject comprising the steps of:
(a) determining the level of tenascin-C in a blood sample from said subject; and
(b) comparing the level of tenascin-C determined in step (a) with one or more reference values.

2. A method of determining the progression of rheumatoid arthritis or the response of rheumatoid arthritis to a particular treatment in a subject comprising the steps of:
(a) determining the level of tenascin-C in a blood sample from said subject; and
(b) comparing the level of tenascin-C determined in step (a) with one or more reference values.

3. A method for determining the appropriate treatment for a subject with rheumatoid arthritis comprising the steps of:
(a) determining the level of tenascin-C in a blood sample from said subject; and
(b) comparing the level of tenascin-C determined in step (a) with one or more reference values
(c) using the results in (b) to determine the most appropriate therapy;
wherein an increase in the level of tenascin-C in a sample from a subject compared to the level in a normal subject, indicates that it is not appropriate to use an anti-TNF drug; or
wherein an increase in the level of tenascin-C in a sample from a subject compared to the level in a normal subject, indicates that it is appropriate to use an anti-IL17 therapy or another none anti-TNF therapy.

4. The method of any preceding claim wherein the rheumatoid arthritis is erosive rheumatoid arthritis.

5. The method of claim 1 for use in determining or diagnosing whether a subject with rheumatoid arthritis has erosive rheumatoid arthritis.

6. The method of any preceding claim wherein an about 50% or more increase in tenascin-C in a sample, compared to a reference level, is diagnostic of rheumatoid arthritis.

7. The method of any of claims 1 to 6 wherein a level of more than 33 ng of tenascin-C per ml of serum is diagnostic of rheumatoid arthritis.

8. The method of any of claims 1 to 6 wherein a level of more than 31 ng of tenascin-C per ml of serum is diagnostic of rheumatoid arthritis.

9. The method of claim 3 wherein the level of tenascin-C in a sample is at least 31 ng/ml of serum.

10. Use of tenascin-C as a blood serum biomarker for rheumatic arthritis.

## Patentansprüche

1. Ein Verfahren für die Bestimmung des Status eines Gelenkrheumatismus bei einem Subjekt, das die folgenden Schritte aufweist:
(a) die Bestimmung des Niveaus von Tenascin-C in einer Blutprobe des besagten Subjekts; und
(b) den Vergleich des Niveaus von Tenascin-C, der in Schritt (a) mit einem oder mehreren Referenzwerten bestimmt wurde.

2. Ein Verfahren für die Bestimmung der Progression des Gelenkrheumatismus oder des Ansprechverhaltens des Gelenkrheumatismus auf eine bestimmte Behandlung bei einem Subjekt, das aus den folgenden Schritten besteht:
(a) die Bestimmung des Niveaus von Tenascin-C in einer Blutprobe des besagten Subjekts; und
(b) den Vergleich des Niveaus von Tenascin-C, der in Schritt (a) mit einem oder mehreren Referenzwerten bestimmt wurde.

3. Ein Verfahren für die Bestimmung der geeigneten Behandlung für ein Subjekt mit Gelenkrheumatismus, das die folgenden Schritte aufweist:
(a) die Bestimmung des Niveaus von Tenascin-C in einer Blutprobe des besagten Subjekts; und
(b) den Vergleich des Niveaus von Tenascin-C, der in Schritt (a) mit einem oder mehreren Referenzwerten bestimmt wurde.
(c) die Verwendung der Resultate in (b) für die Bestimmung der geeignetsten Therapie;
wobei eine Zunahme des Niveaus von Tenascin-C in einer Probe von einem Subjekt im Vergleich zum Niveau bei einem normalen Subjekt anzeigt, dass der Einsatz eines Anti-TNF-Medikaments nicht angemessen ist; oder
wobei eine Zunahme des Niveaus von Tenascin-C in einer Probe von einem Subjekt im Vergleich zum Grad bei einem normalen Subjekt anzeigt, dass der Einsatz einer Anti-IL17-Therapie oder einer anderen Nicht-Anti-TNF-Therapie angemessen ist.

4. Das Verfahren gemäß eines der vorhergehenden Ansprüche, wobei der Gelenkrheumatismus ein erosiver Gelenkrheumatismus ist.

5. Das Verfahren gemäß Anspruch 1 für die Verwendung bei der Bestimmung oder Diagnose, ob das Subjekt mit Gelenkrheumatismus einen erosiven Gelenkrheumatismus zeigt.

6. Das Verfahren gemäß eines der vorhergehenden Ansprüche, wobei eine Zunahme von ungefähr 50% oder mehr von Tenascin-C in einer Probe, im Vergleich zu einem Referenzniveau, den Gelenkrheumatismus diagnostiziert.

7. Das Verfahren gemäß eines der Ansprüche 1 bis 6, wobei ein Niveau von mehr als 33 ng Tenascin-C pro ml Serum den Gelenkrheumatismus diagnostiziert.

8. Das Verfahren gemäß eines der Ansprüche 1 bis 6, wobei ein Niveau von mehr als 31 ng Tenascin-C pro ml Serum den Gelenkrheumatismus diagnostiziert.

9. Das Verfahren gemäß Anspruch 3, wobei das Niveau von Tenascin-C in einer Probe mindestens 31 ng/ml des Serums beträgt.

10. Die Verwendung von Tenascin-C als Blutserum-Biomarker für Gelenkrheumatismus.

## Revendications

1. Un procédé de détermination de l'état de polyarthrite rhumatoïde d'un sujet comprenant les opérations suivantes :
(a) la détermination du niveau de ténascine-C dans un échantillon sanguin provenant dudit sujet, et
(b) la comparaison du niveau de ténascine-C déterminé à l'opération (a) à une ou plusieurs valeurs de référence.

2. Un procédé de détermination de la progression de polyarthrite rhumatoïde ou de la réponse de polyarthrite rhumatoïde à un traitement particulier chez un sujet comprenant les opérations suivantes :
(a) la détermination du niveau de ténascine-C dans un échantillon sanguin provenant dudit sujet, et
(b) la comparaison du niveau de ténascine-C déterminé à l'opération (a) à une ou plusieurs valeurs de référence.

3. Un procédé de détermination du traitement approprié pour un sujet souffrant de polyarthrite rhumatoïde comprenant les opérations suivantes :
(a) la détermination du niveau de ténascine-C dans un échantillon sanguin provenant dudit sujet, et
(b) la comparaison du niveau de ténascine-C déterminé à l'opération (a) à une ou plusieurs valeurs de référence,
l'utilisation des résultats de (b) de façon à déterminer la thérapie la plus appropriée,
où une augmentation du niveau de ténascine-C dans un échantillon provenant d'un sujet en comparaison d'un niveau chez un sujet normal indique qu'il n'est pas approprié d'utiliser un médicament anti-TNF, ou
où une augmentation du niveau de ténascine-C dans un échantillon provenant d'un sujet en comparaison d'un niveau chez un sujet normal indique qu'il est approprié d'utiliser une thérapie anti-IL17 ou un autre thérapie non anti-TNF.

4. Le procédé selon l'une quelconque des Revendications précédentes où la polyarthrite rhumatoïde est une polyarthrite rhumatoïde érosive.

5. Le procédé selon la Revendication 1 destiné à une utilisation dans la détermination ou le diagnostic si un sujet souffrant de polyarthrite rhumatoïde présente une polyarthrite rhumatoïde érosive.

6. Le procédé selon l'une quelconque des Revendications précédentes où une augmentation d'environ 50% ou plus de ténascine-C dans un échantillon, en comparaison d'un niveau de référence, est un diagnostic de polyarthrite rhumatoïde.

7. Le procédé selon l'une quelconque des Revendications 1 à 6 où un niveau de plus de 33 ng de ténascine-C par ml de sérum est un diagnostic de polyarthrite rhumatoïde.

8. Le procédé selon l'une quelconque des Revendications 1 à 6 où un niveau de plus de 31 ng de ténascine-C par ml de sérum est un diagnostic de polyarthrite rhumatoïde.

9. Le procédé selon la Revendication 3 où le niveau de ténascine-C dans un échantillon est au moins de 31 ng/ml de sérum.

10. L'utilisation de ténascine-C en tant que biomarqueur de sérum sanguin pour la polyarthrite rhumatoïde.
